# EUROPEAN PATENT APPLICATION

(11) **EP 2 022 508 A1**
(43) Date of publication of application: **11.02.2009**
(21) Application number: 07015538.7
(22) Date of filing: 07.08.2007
(51) Int. Cl.: A61K 49/18

(54) **Production of targeted MRI probes by biocompatible coupling of macromolecules with charged nanoparticles**

(71) Applicant: Charité-Universitätsmedizin Berlin, 10117 Berlin (DE)
(72) Inventor: Schellenberger, Eyk, D-10115 Berlin (DE); Wagner, Susanne, D-15831 Mahlow (DE); Schnorr, Jörg, D-10249 Berlin (DE)
(74) Representative: Engelhard, Markus

(57) **Abstract**

The present invention relates to the production of marker particles, which are especially useful for target-specific molecular magnetic resonance imaging (MRI), using charged iron oxide nanoparticles and inversely charged peptides covalently linked to macromolecules, which are capable of binding to a specific type of cells.

## Description

The present invention relates to the production of marker particles, which are especially useful for target-specific molecular magnetic resonance imaging (MRI), using charged iron oxide nanoparticles and inversely charged peptides covalently linked to macromolecules, which are capable of binding to a specific type of cells.

For about two decades superparamagnetic iron oxide nanoparticles have been developed for magnetic resonance imaging (MRI). Because of their excellent detectability due to strong negative contrast effects in T2 and T2* weighted MR imaging sequences iron oxide nanoparticles comprise also excellent properties for the design of targeted probes for diagnostic imaging of specific biological functions (Corot C. et al., Adv Drug Deliv Rev 58 (2006) 1471-1504).
Due to their magnetic properties iron oxide nanoparticles have a natural tendency to aggregate. To keep particles apart two main ways of particle stabilization have been developed: For steric stabilization particles are coated with a thick layer of macromolecules, which keep the iron oxide cores sterically apart. Because of the necessity of a minimal thickness of the coating, steric stabilization works down to a hydrodynamic diameter of about 20 nm. In contrast, electrostatic stabilization is based on the repulsion caused by positive or negative charged surface coatings (Corot C. et al., Adv Drug Deliv Rev 58 (2006) 1471-1504). Since this stabilization is independent of the coating thickness, the design of even smaller electrostatic stabilized iron oxide particles is possible e.g. down to about 3 nm for very small iron oxide particles (VSOP, unpublished Data of Ferropharm GmbH). VSOP-C184, for example, which have successfully passed clinical evaluation phase 1, have a hydrodynamic diameter of approximately 7.5 nm. These extremely small but potent nanoparticles open up the possibility to develop iron oxide nanoparticles for molecular imaging well below 20 nm.
VSOP are citrate-monomer coated iron oxide nanoparticles that successfully passed clinical evaluation phase I (Taupitz M. et al., Invest Radiol 39 (2004) 394-405) and undergo currently clinical evaluation phase II for MR angiography. The coupling of large molecules like proteins to these particles (e.g. in order to create a targeted probe) is challenging since the coating citrate monomers are bound to the iron oxide core by complexation rather than by covalent bonds.
Programmed cell death or apoptosis is an essential biological feature, which is as important as mitosis for the development and tissue homeostasis of multicellular organisms (Kerr J.F. et al., Br J Cancer 26 (1972) 239-257). In terms of clinical significance altered apoptosis is fundamental to countless diseases e.g. cancer and cardiovascular diseases. Thus, the development of molecular imaging probes for apoptosis with many potential applications is especially attractive to demonstrate the feasibility for design of protein-coupled imaging probes. So far, based on annexin (Anx) V (Reutelingsperger C.P. et al., Eur J Biochem 173 (1988) 171-178), a standard *in vitro* marker for apoptosis, several probes for *in vivo* imaging with nuclear (Hofstra L. et al., Lancet 356 (2000) 209-212; Kietselaer B.L. et al., N Engl J Med 350 (2004) 1472-1473), PET (positron emission tomography) (Collingridge D.R. et al., Br J Cancer 89 (2003) 1327-1333), NIRF (near infrared fluorescence) (Schellenberger E.A. et al., Neoplasia 5 (2003) 187-192), and MR imaging (Schellenberger E.A. et al., Bioconjug Chem 15 (2004) 1062-1067; Sosnovik D.E. et al., Magn Reson Med 54 (2005) 718-724) have been developed. For instance, the MRI probe Annexin V-CLIO has a hydrodynamic diameter of about 50 nm.

Accordingly, it was an object of the present invention to provide for an easy, cost effective, and biocompatible method of coupling macromolecules, e.g. polypeptides such as annexin V, to iron oxide nanoparticles.
It was also an object of the present invention to provide for a method of producing targeted probes, especially for use in MRI, which are significantly smaller than previously described marker particles and also have very sensitive detectability.

The objects of the present invention are solved by a method of producing a marker particle, especially for magnetic resonance imaging (MRI), said method comprising
covalently linking a macromolecule to a charged peptide, and
electrostatically coupling said charged peptide to an iron oxide particle,
wherein said iron oxide particle is coated by an inversely charged shell.

In one embodiment of the present invention said iron oxide particle has a diameter < 1000 nm.

In one embodiment said charged shell is an anionic shell.

In one embodiment said anionic shell is formed by a plurality of citrate monomers.

In one embodiment said iron oxide particle is a VSOP (very small iron oxide particle) having a diameter ≤ 20 nm, preferably ≤ 15 nm, more preferably ≤ 10 nm.

In one embodiment said charged peptide comprises more than 10 amino acids, preferably more than 20 amino acids.

In one embodiment said charged peptide is a polycationic peptide.

In one embodiment said polycationic peptide has a percentage of positively charged amino acids of more than 45%, preferably of more than 60%.

In one embodiment of the present invention said polycationic peptide is protamine.

In one embodiment said macromolecule is a polypeptide.

In one embodiment said polypeptide is capable to specifically bind to a certain cell type.

In one embodiment said cell type is selected from the group comprising apoptotic cells, mitotic cells, tumor cells, activated macrophages, activated endothelial cells, bacterial cells, and degenerative cells.

In one embodiment said cell type is apoptotic cells, wherein, preferably, said polypeptide is annexin V.

In one embodiment said covalent linking is based on a bond selected from the group of disulfide bond, amide bond, and peptide bond.

In one embodiment said covalent linking is based on a disulfide bond, wherein, preferably, said charged peptide, specially said protamine, is activated for forming said disulfide bond by reaction with SPDP (N-Succinimidyl 3-(2-Pyridyldithio)propionat).

In one embodiment said disulfide group is formed by a thiol group introduced by site-directed mutagenesis.

In one embodiment said covalent linking is based on a peptide bond, wherein, preferably, said peptide bond is obtained by translating a chimera of said polypeptide and said charged peptide from the same mRNA in *E. coli*.

In one embodiment said marker particle has a diameter ≤ 40 nm, preferably ≤ 30 nm, more preferably ≤ 20 nm.

The objects of the present invention are also solved by a marker particle produced by the method according to the present invention.

The objects of the present invention are also solved by a marker particle, especially for magnetic resonance imaging (MRI), comprising
an iron oxide particle,
a charged shell coating said iron oxide particle,
at least one inversely charged peptide electrostatically coupled to said charged shell, and
a macromolecule covalently linked to said charged peptide.

In one embodiment said iron oxide particle has a diameter < 1000 nm.

In one embodiment said charged shell is an anionic shell.

In one embodiment said anionic shell is formed by a plurality of citrate monomers.

In one embodiment said iron oxide particle is a VSOP (very small iron oxide particle) having a diameter ≤ 20 nm, preferably ≤ 15 nm, more preferably ≤ 10 nm.

In one embodiment said charged peptide comprises more than 10 amino acids, preferably more than 20 amino acids.

In one embodiment said charged peptide is a polycationic peptide.

In one embodiment said polycationic peptide has a percentage of positively charged amino acids of more than 45%, preferably of more than 60%.

In one embodiment said polycationic peptide is protamine.

In one embodiment said macromolecule is a polypeptide.

In one embodiment said polypeptide is capable to specifically bind to a certain cell type.

In one embodiment said cell type is selected from the group comprising apoptotic cells, mitotic cells, tumor cells, activated macrophages, activated endothelial cells, bacterial cells, and degenerative cells.

In one embodiment said cell type is apoptotic cells, wherein, preferably, said polypeptide is annexin V.

In one embodiment said covalent linking is based on a bond selected from the group of disulfide bond, amide bond, peptide bond.

In one embodiment said covalent linking is based on a disulfide bond, wherein, preferably, said charged peptide, specially said protamine, is activated for forming said disulfide bond by reaction with SPDP (N-Succinimidyl 3-(2-Pyridyldithio) propionat).

In one embodiment said disulfide bond is formed by a thiol group introduced by site-directed mutagenesis.

In one embodiment said covalent linking is based on a peptide bond, wherein, preferably, said peptide bond is obtained by translating a chimera of said polypeptide and said charged peptide from the same mRNA in *E. coli.*

In one embodiment said marker particle has a diameter ≤ 40 nm, preferably ≤ 30 nm, more preferably ≤ 20 nm.

The objects of the present invention are also solved by the use of said marker particle for MRI.

The term "biocompatible coupling", as used herein, is meant to refer to way of coupling two substrates or molecules by using coupling agents or cross linkers that do not have toxic or injurious effects on biological systems.

The term "inversely charged", as used herein, is meant to refer to the fact that a positively or negatively charged peptide interacts with an inversely, i.e. negatively or positively, charged shell, and a negatively or positively charged shell interacts with an inversely, i.e. positively or negatively, charged peptide.

The term "marker particle", is used herein synonymously with the term "probe" and is meant to refer to a particle suitable for detection by diagnostic imaging techniques, especially MRI.
The term "targeted probe" is meant to refer to a marker particle or probe, which is capable of selectively binding to a specific type of cell, therefore allowing diagnostic imaging of specific biological functions.

The term "translating a chimera", as used herein, is meant to refer to the translation of a single mRNA, coding for both the polypeptide and charged peptide, into the corresponding fusion protein of both.

The term "polypeptide ... capable to specifically bind to a certain cell type" is meant to refer to a polypeptide, which can bind to a specific type of cell or to specific extracellular compounds, e.g. extracellular matrix proteins, but not to others. An example for such a polypeptide is annexin V, which binds with high preference to apoptotic cells.

The term "polycationic peptide" is meant to refer to a highly positively charged peptide comprising more then 10 amino acids and having a percentage of positively charged amino acids of more than 45%.

The inventors have surprisingly found that using charged peptides, such as polycationic protamine, in order to couple macromolecules, e.g. target-specific polypeptides such as annexin V, to the charged surface of iron oxide nanoparticles provides for a relatively simple and cost effective method of producing targeted marker particles, especially for MRI.
The use of electrostatically stabilized VSOP (very small iron oxide particles) having a diameter down to about 3 nm and a negatively charged surface formed by a citrate shell allows the production of targeted probes below 20 nm. This is significantly smaller than previously described particles with advantages like increased bioavailability in the target tissue and increased blood half-life due to decreased phagocytosis. In addition, the superparamagnetic VSOP are detectable by MRI with high sensitivity.
Furthermore, all compounds are highly biocompatible, since protamine is clinically approved and annexin V and VSOPs are in clinical evaluation.
The method according to the present invention can be applied to any other protein besides annexin V, since the electrostatic linker agent SPDP-protamine can be coupled to any protein that naturally contains a chemically reactive thiol group or that was thiolated prior to the reaction. Additionally, the coupling method could be applied to other charged surfaces, maybe even to charged electrodes.
In an alternative embodiment of the present invention the coupling method is further simplified to a "mix-and-ready" procedure by translating a chimera of a polypeptide (e.g. annexin V) and a charged peptide (e.g. protamine) from the same mRNA in *E. coli* and mixing the purified chimera with the anionic iron oxide particles.

Reference is now made to the figures, wherein
**Figure 1** shows a schematic description of the synthesis of a macromolecule-VSOP, e.g. annexin V-VSOP (Anx-VSOP).
**Figure 2** shows a SDS-page protein gel of synthesized protamine-annexin V.
**Figure 3a** shows a molecular model of Anx-VSOP. **Figure3b** shows a transmission electron microscopy picture (TEM) of VSOPs. **Figure 3c** shows the proportion of Anx-VSOP in comparison to an IgG antibody **(****Figure 3d****)** and to an annexin V-CLIO particle **(****Figure 3e****).**
**Figure 4** shows a graph of the statistics of size measurements of Anx-VSOP.
**Figure 5** shows sigmoidal dose-response curves demonstrating the relative binding of Anx-VSOP to apoptotic cells.
**Figure 6** shows an image of *in vitro* MRI using Anx-VSOP

The invention is now further described by reference to the following examples which are intended to illustrate, not to limit the scope of the invention.

### Example 1

### Synthesis of Anx-VSOP

SPDP (N-succinimidyl 3-(2-pyridyldithio) propionate) and the BCA-protein assay Kit were purchased from Pierce. VSOPs (VSOP-C200, Very small iron oxide particles) were provided by Ferropharm GmbH, Germany and Cys-annexin V by PharmaTarget, The Netherlands.
Sephadex G15 gel was purchased from Amersham Biosciences, BioGel P6 was purchased from BioRad. FITC-annexin (Anx) V was obtained from Clontech. All other chemicals inclusive protamine (from salmon) and the SDS Gel Preparation Kit were purchased from Sigma-Aldrich.

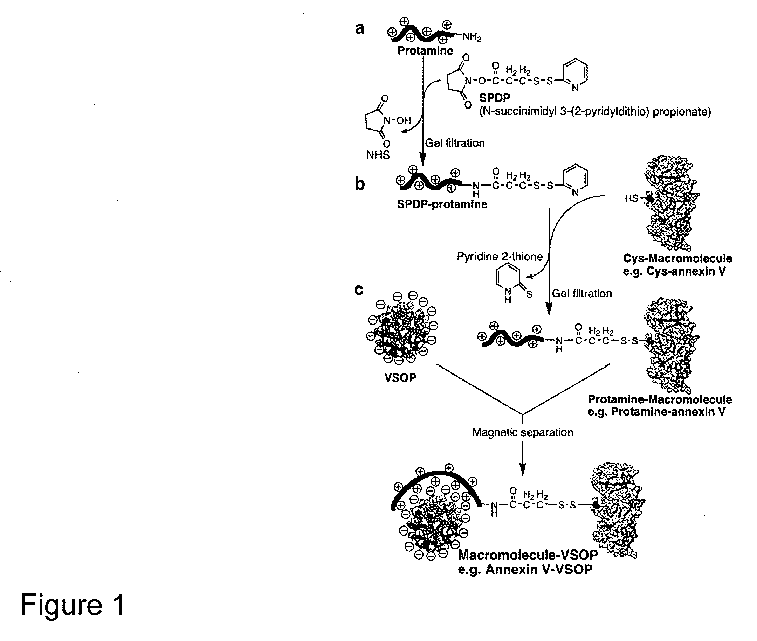

The synthesis of Anx-VSOP was done in 3 main steps (a, b, and c in **Figure 1****).**

To activate protamine for reaction with thiol groups 900 µl protamine (2.1 g/l in 50 mM NaHPO₂, pH 8.0) was incubated with 22.5 µl SPDP (100 mM in DMSO) overnight at 4 °C. Low molecular impurities were removed by double gelfiltration using a Sephadex G15 column (in PBS, pH 7.4). The protein concentration of the resulting SPDP-protamine was determined by a BCA-protein assay. The level of SPDP-modification of protamine was determined spectrophotometrically by measuring the extinction (E₃₄₃= 8,080 M⁻¹cm⁻¹) before and after treatment with DTT according to the SPDP data sheet (Pierce).

Potential cystein-dimers of cys-annexin V protein (3.8 g/l) were reduced by treatment with DTT (10 mM final concentration) for 90 min at 37 °C. To remove DTT the solution of reduced cys-annexin V was dialyzed for 24 h at 4 °C (25 mM HEPES, 140 mM NaCl, 1 mM EDTA, pH 7.4).

To synthesize protamine-annexin V 170 µl cys-annexin V, which is a variant of human annexin V modified by a point mutation to exchange the N-terminal glutamine for a cysteine in order to introduce a solitary thiol-coupling site (Prinzen, L. et al., Nano Lett 7 (2007) 93-100) were then reacted with SPDP-protamine (755 µl, SPDP-concentration 0.14 mM) with addition of NaCl (final concentration 500 mM) overnight at 4 °C. Since this thiol is located opposite to the membrane-binding site of annexin V, linking the iron oxide nanoparticle to this thiol ensures the preservation of the binding affinity to the plasma membrane of apoptotic cells. Unreacted SPDP-protamine was removed from the resulting protamine-annexin V by gelfiltration with a BioGel P6 column (in 20 mM NaHCO₃, 140 mM NaCl, pH 8.1). As control experiment for unspecific binding of protamine to cys-annexin, non-activated protamine was incubated with cys-annexin V and filtrated under the same conditions.
Protamine-annexin V concentration was 0.39 g/l as determined by a BCA-protein assay. Cys-annexin V, protamine-annexin V and the protamine-annexin V mixture of the control experiment were analyzed by standard SDS page gel electrophoresis with and without reducing mercaptoethanol.

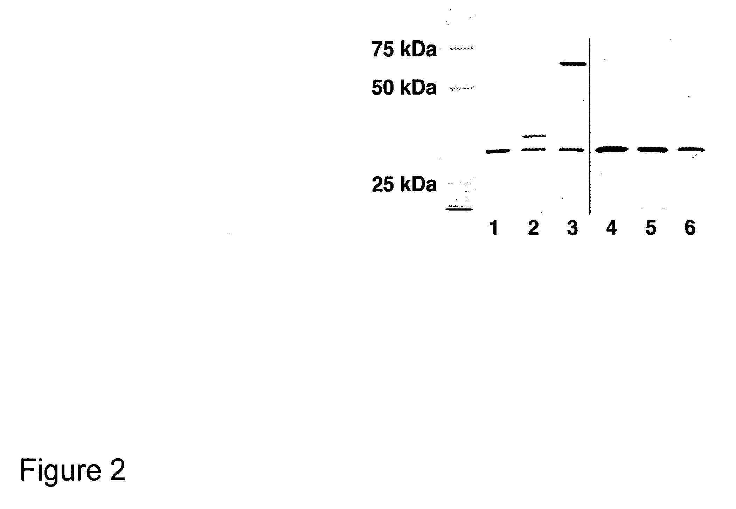

**Figure 2** shows a SDS page protein gel of protamine-annexin V. Lane (a) shows the 35 kDa protein cys-annexin V in comparison to the reaction product protamine-annexin V and unreacted cys-annexin V (b). Lane (c) shows the result of a control experiment with non-activated protamine to show that the coupling of protamine-annexin V is due to the disulfide bond and not to unspecific electrostatic absorption. Cys-annexin V monomers and dimers (70 kDa) are visible. Lanes (d - f) display the separation of the same samples, but under conditions, which break the disulfide bridge between cys-annexin and protamine. Lane (e) reveals the reduced cys-annexin without coupled protamine.

For the final product 753 µl protamine-annexin V and 37 µl VSOP (49 mM iron concentration) were incubated overnight at 4°C. Unbound annexin V or protamine-annexin V was separated from Anx-VSOP by magnetic separation using MACS LS columns (Miltenyi Biotec, elution buffer 20 mM NaHCO₃, 140 mM NaCl, pH 8.2).

An interesting aspect is that reaction step I and II and the magnetic separation step III **(****Figure 1****)** could maybe completely avoided by expression and purification of a protamine-annexin V chimera in *E. coli* and mixing said chimera with the VSOPs. The synthesis of Anx-VSOP could therefore be a simple "mix-and-ready" procedure.

### Example 2

### Physical characterization and molecular modeling of Anx-VSOP

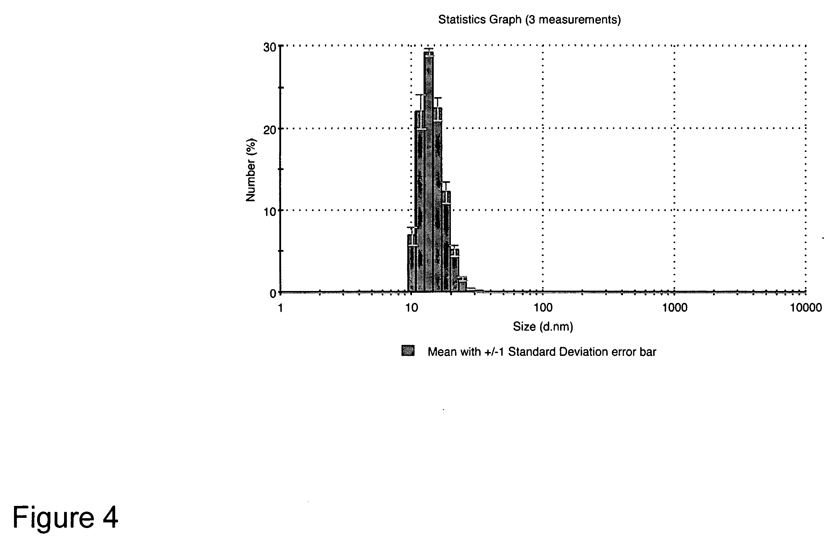

The size of the resulting Anx-VSOP nanoparticles was determined by dynamic light scattering (Zetasizer Nano ZS, Malvern Instruments) **(****Figure 4****)** and R1 and R2 relaxivities were measured with a MR spectrometer at 0.94 T (Bruker, Minispec MQ 40).
The iron concentration was determined spectrophotometrically (Shen T. et al., Magn Reson Med 29 (1993) 599-604) and the nanoparticle number calculated assuming 2600 Fe atoms per nanoparticle (data provided by Ferropharm GmbH). The number of annexin V molecules bound per VSOP particle was determined to be 5 by measuring the protein concentration with a BCA-assay. Due to their relative large iron oxide core (∼ 5 nm) Anx-VSOP comprise a high R2 relaxivity of 70 mM-1s-1 (per Fe) or 180,000 mM-1s-1 (per particle) indicating very sensitive detectability in MRI.
The results are summarized in **Table 1.**

**Table 1 Properties of Anx-VSOP**

| | |
|---|---|
| size VSOP C200 [nm] | 8.7 ±2.0 |
| size iron oxide core VSOP C200 [nm]* | 5 ±0.4 |
| size Anx-VSOP [nm] | 14.6 ±3.2 |
| moles of annexin V per VSOP [nm] | ∼5 |
| R1 (Fe) [mM⁻¹s⁻¹] | 22 |
| R2 (Fe) [mM⁻¹s⁻¹] | 70 |
| R1-particle [mM⁻¹ s⁻¹]** | 57,000 |
| R2-particle [mM⁻¹ s⁻¹]** | 180,000 |

| | |
|---|---|
| * Data provided by Ferropharm GmbH ** Relaxivity for nanoparticle-concentration with 2600 iron atoms per nanoparticle | |

Molecular modeling was performed with PyMOL Molecular Graphics System (DeLano W.L., DeLano Scientific (2002) USA). The crystal structure of annexin V (Lewit-Bentley A. et al., Eur J Biochem 210 (1992) 73-77) was modified by exchanging the N-terminal glutamine for a cysteine according to the point mutation for creation of a single thiol-coupling site. This cysteine was connected with the N-terminal end of protamine (Ando T. et al., Int J Protein Res 1 (1969) 221-224) via the cross linker SPDP. The iron oxide core size of VSOP-C200 with 5 ±0.4 nm was measured by transmission electron microscopy (TEM). The approximate number of 75 citrate molecules per nanoparticle core was determined by HPLC.

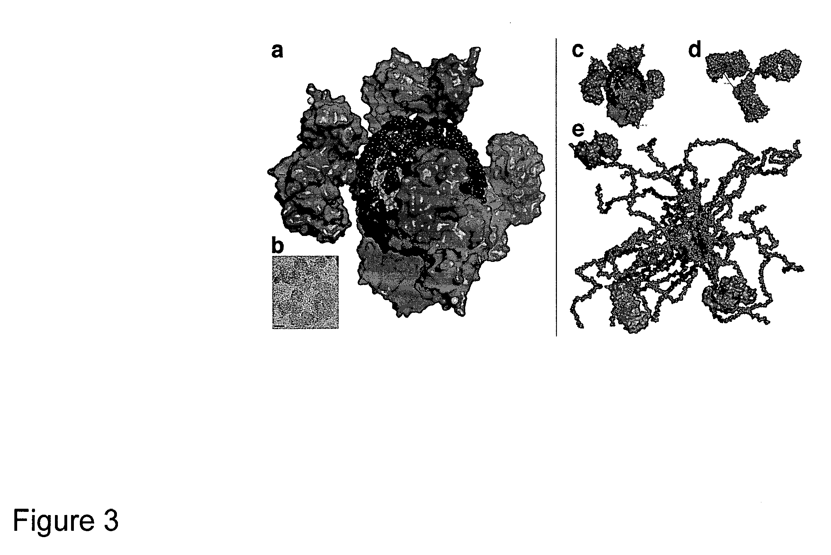

**Figure 3** shows as the result of molecular modeling of the putative structure of Anx-VSOP. The model was built by creating a 5 nm magnetite core, coated and electrostatically stabilized by about 75 citrate molecules (data including TEM image provided by Ferropharm). Attached to this VSOP-model are 5 protamine-annexin V molecules. The overall diameter is about 14 nm matching the size measurement by dynamic light scattering very well **(Table 1).**

### Example 3

### Affinity of Anx-VSOP for apoptotic cells and MRI

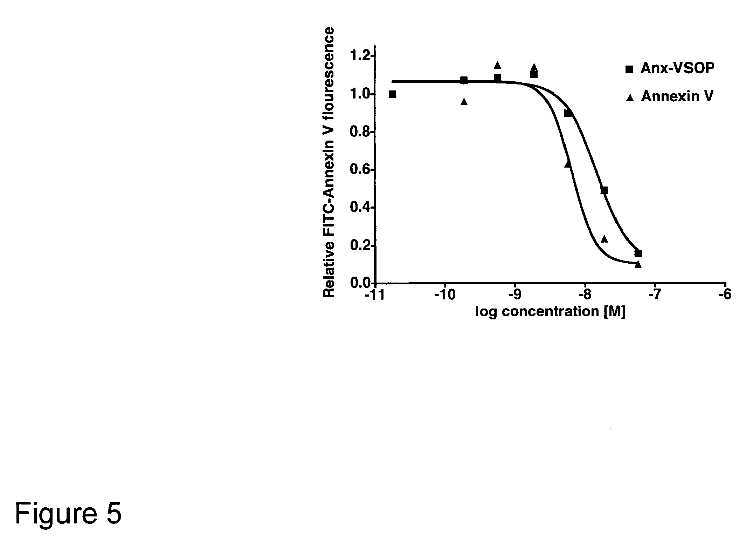

In order to assess the binding affinity of Anx-VSOP to apoptotic Jurkat T cells the displacement of annexin-FITC by Anx-VSOP in comparison to native annexin V was analyzed.
Jurkat T cells (Clone E6-1, ATCC #TIB-152) were grown according to ATCC protocols. Apoptosis was induced by the addition of camptothecin to the culture medium (7 µM final concentration), and induction was verified by staining with annexin V-FITC (Clontech). All experiments were performed in annexin V binding buffer (BB) containing 1.3 mM CaCl₂, 10 mM HEPES, 150 mM NaCl, 5 mM KCI, 1 mM MgCl₂, pH 7.4. After washing the cells twice, they were incubated with increasing concentrations of Anx-VSOP or annexin V for 5 min at room temperature (∼50,000 camptothecin treated cells in 500 µl BB per test tube). Then FITC-annexin V (0.03 µg) was added and incubated for additional 10 min at room temperature. Binding of FITC-annexin V to the apoptotic cells was determined from the medians of the highest peaks obtained by flow cytometry using a FACS-Calibur cytometer (Becton Dickinson) according the manufacturer's manual (Schellenberger E.A. et al., Neoplasia 5 (2003) 187-192). IC₅₀'s of Anx-VSOP and annexin V were determined by the ability to displace FITC-annexin V. The data were fitted to sigmoidal dose-response curves **(****Figure 5****)** to obtain IC₅₀'s, values of the Hill coefficient (n) and the correlation coefficient (R²), indicating the goodness of fit, using Prism 4 (GraphPad Software, San Diego, CA).
The sigmoid dose-response-analysis shown in **Figure 5** revealed that Anx-VSOP replaced annexin V-FITC with an IC₅₀ of 15 nM (95 % confidence interval 11 to 20 nM, R² = 0.99, Hill coefficient 1.8), which was only about twice as high as the IC₅₀ of unmodified annexin V protein with 6,5 nM (95 % confidence interval 3.9 to 11 nM, R² = 0.96, Hill coefficient 2.5). These results confirmed that the binding properties of cys-annexin were not substantially affected by the coupling procedure.

For MRI, Jurkat T cells where incubated with Anx-VSOP (1 µg Fe /ml) for 10-15 minutes and washed twice with BB. Cell pellets in 300 µl tubes were prepared by centrifugation (Schellenberger E.A. et al., Bioconjug Chem 15 (2004) 1062-1067) and imaged on a human 1.5T MR scanner (Siemens Sonata). Transverse relaxation times (T2's) were determined by fitting signal intensity data (Gradient echo sequence, TE = 15,5 - 186 ms, TR = 3000 ms) to the spin echo signal intensity equation (Schellenberger E.A. et al., Bioconjug Chem 15 (2004) 1062-1067). A T2* weighted image was also taken at TE= 15, TR= 200 and flip angle 40.

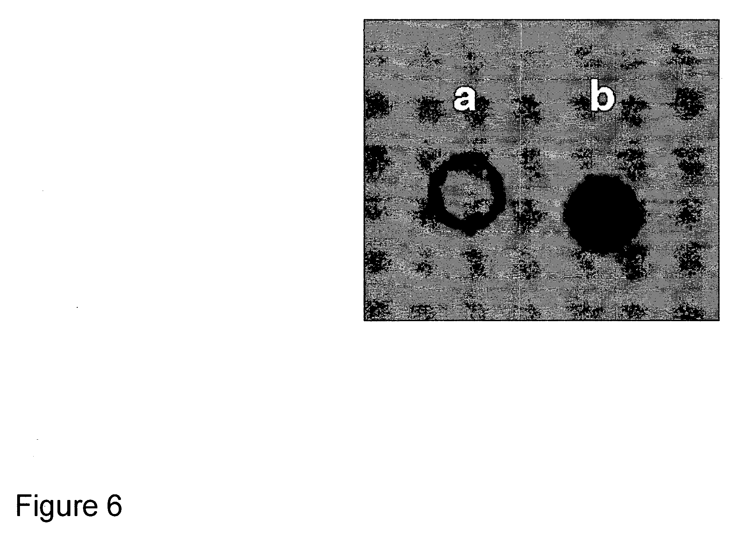

**Figure 6** shows an *in vitro* MRI image of two 300 µl PCR tubes containing cell pellets with 7 % apoptotic cells in (a) and 26 % apoptotic cells in (b).

## Claims

1. A method of producing a marker particle, especially for magnetic resonance imaging (MRI), said method comprising
covalently linking a macromolecule to a charged peptide, and
electrostatically coupling said charged peptide to an iron oxide particle,
wherein said iron oxide particle is coated by an inversely charged shell.

2. The method according to claim 1, wherein said iron oxide particle has a diameter < 1000 nm.

3. The method according to any of the foregoing claims, wherein said charged shell is an anionic shell.

4. The method according to claim 3, wherein said anionic shell is formed by a plurality of citrate monomers.

5. The method according to any of the foregoing claims, wherein said iron oxide particle is a VSOP (very small iron oxide particle) having a diameter ≤ 20 nm, preferably ≤ 15 nm, more preferably ≤ 10 nm.

6. The method according to any of the forgoing claims, wherein said charged peptide comprises more than 10 amino acids, preferably more than 20 amino acids.

7. The method according to any of the foregoing claims, wherein said charged peptide is a polycationic peptide.

8. The method according claim 7, wherein said polycationic peptide has a percentage of positively charged amino acids of more than 45%, preferably of more than 60%.

9. The method according to claim 7, wherein said polycationic peptide is protamine.

10. The method according to any of the foregoing claims, wherein said macromolecule is a polypeptide.

11. The method according to claim 10, wherein said polypeptide is capable to specifically bind to a certain cell type.

12. The method according to claim 11, wherein said cell type is selected from the group comprising apoptotic cells, mitotic cells, tumor cells, activated macrophages, activated endothelial cells, bacterial cells, and degenerative cells.

13. The method according to claim 12, wherein said cell type is apoptotic cells.

14. The method according to claim 13, wherein said polypeptide is annexin V.

15. The method according to any of the foregoing claims, wherein said covalent linking is based on a bond selected from the group of disulfide bond, amide bond, and peptide bond.

16. The method according to claim 15, wherein said covalent linking is based on a disulfide bond.

17. The method according to claim 16, wherein said charged peptide, specially said protamine, is activated for forming said disulfide bond by reaction with SPDP (N-Succinimidyl 3-(2-Pyridyldithio)propionat).

18. The method according to claim 16, wherein said disulfide bond is formed by a thiol group introduced by site-directed mutagenesis.

19. The method according to claim 15, wherein said covalent linking is based on a peptide bond.

20. The method according to claim 19, wherein said peptide bond is obtained by translating a chimera of said polypeptide and said charged peptide from the same mRNA in *E. coli*.

21. The method according to any of the foregoing claims, wherein said marker particle has a diameter ≤ 40 nm, preferably ≤ 30 nm, more preferably ≤ 20 nm.

22. A marker particle produced by the method according to any of the claims 1-21.

23. A marker particle, especially for magnetic resonance imaging (MRI), comprising
an iron oxide particle,
a charged shell coating said iron oxide particle,
at least one inversely charged peptide electrostatically coupled to said charged shell, and
a macromolecule covalently linked to said charged peptide.

24. The marker particle according to claim 23, wherein said iron oxide particle has a diameter < 1000 nm.

25. The marker particle according to any of the foregoing claims 23 - 24, wherein said charged shell is an anionic shell.

26. The marker particle according to claim 25, wherein said anionic shell is formed by a plurality of citrate monomers.

27. The marker particle according to any of the foregoing claims 23 - 26, wherein said iron oxide particle is a VSOP (very small iron oxide particle) having a diameter ≤ 20 nm, preferably ≤ 15 nm, more preferably ≤ 10 nm.

28. The marker particle according to any of the forgoing claims 23 - 27, wherein said charged peptide comprises more than 10 amino acids, preferably more than 20 amino acids.

29. The marker particle according to any of the foregoing claims 23 - 28, wherein said charged peptide is a polycationic peptide.

30. The marker particle according to claim 29, wherein said polycationic peptide has a percentage of positively charged amino acids of more than 45%, preferably of more than 60%.

31. The marker particle according to claim 29, wherein said polycationic peptide is protamine.

32. The marker particle according to any of the foregoing claims 23 - 31, wherein said macromolecule is a polypeptide.

33. The marker particle according to claim 32, wherein said polypeptide is capable to specifically bind to a certain cell type.

34. The marker particle according to claim 33, wherein said cell type is selected from the group comprising apoptotic cells, mitotic cells, tumor cells, activated macrophages, activated endothelial cells, bacterial cells, and degenerative cells.

35. The marker particle according to claim 34, wherein said cell type is apoptotic cells.

36. The marker particle according to claim 35, wherein said polypeptide is annexin V.

37. The marker particle according to any of the foregoing claims 23 - 36, wherein said covalent linking is based on a bond selected from the group of disulfide bond, amide bond, peptide bond.

38. The marker particle according to claim 37, wherein said covalent linking is based on a disulfide bond.

39. The marker particle according to claim 38, wherein said charged peptide, specially said protamine, is activated for forming said disulfide bond by reaction with SPDP (N-Succinimidyl 3-(2-Pyridyldithio) propionat).

40. The marker particle according to claim 38, wherein said disulfide bond is formed by a thiol group introduced by site-directed mutagenesis.

41. The marker particle according to claim 37, wherein said covalent linking is based on a peptide bond.

42. The marker particle according to claim 41, wherein said peptide bond is obtained by translating a chimera of said polypeptide and said charged peptide from the same mRNA in *E. coli*.

43. The marker particle according to any of the foregoing claims 23 - 42 having a diameter ≤ 40 nm, preferably ≤ 30 nm, more preferably ≤ 20 nm

44. The use of a marker particle according to any of claims 22 - 43 for MRI.
